(19)
Europäisches
Patentamt
European
Patent Office
Office européen
des brevets

(11) **EP 2 153 830 A1**

(12) **EUROPEAN PATENT APPLICATION**

(43) Date of publication:
**17.02.2010 Bulletin 2010/07**

(51) Int Cl.:
*A61K 31/343* (2006.01)   *A61P 9/10* (2006.01)

(21) Application number: **08290761.9**

(22) Date of filing: **07.08.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicant: **Sanofi-Aventis**
**75013 Paris (FR)**

(72) Inventors:
 • **Gaudin, Christophe**
  **75013 Paris (FR)**

 • **Hamdani, Nacéra**
  **75013 Paris (FR)**
 • **Radzik, David**
  **75013 Paris (FR)**
 • **Van Eickels, Martin**
  **75013 Paris (FR)**

(74) Representative: **Morel-Pécheux, Muriel et al**
**Sanofi-Aventis**
**Département Brevets**
**174 avenue de France**
**75013 Paris (FR)**

(54) **Use of dronedarone for the preparation of a medicament intended for the prevention of stroke or transient ischemic attack**

(57) Use of dronedarone for the preparation of a medicament intended for the prevention of stroke or transient ischemic attack.

EP 2 153 830 A1

**Description**

The instant invention relates to the use of dronedarone for the preparation of a medicament intended for the prevention of stroke or transient ischemic attack.

2-n-butyl-3-[4-(3-di-n-butylaminopropoxy)benzoyl]-5-méthylsulfonamidobenzofuranne or dronedarone and its pharmaceutically acceptable salts are described in European patent EP 0 471 609 B1.

Dronedarone is a multi-channel blocker that affects calcium, potassium and sodium channels and has anti-adrenergic properties.

Dronedarone is an anti-arrhythmic agent for the treatment of patients with a history of atrial fibrillation or atrial flutter.

Atrial fibrillation (AF) affects about 2.3 million people in North America and 4.5 million people in the European Union and is emerging as a growing public health concern because of the aging of the population

AF is a condition in which the upper chambers of the heart beat in an uncoordinated and disorganized fashion, resulting in a very irregular and fast rhythm (i.e., an irregularly, irregular heartbeat). When blood is not completely pumped out of the heart's chambers, it can pool and clot. If a blood clot forms in the atria, exits the heart and blocks an artery in the brain, a stroke results. Consequently, about 15 percent of strokes result from AF. But stroke can also complicate other conditions like for example hypertension. Also hemorrhagic strokes can be a complication of treatment with an anticoagulant prescribed to prevent the formation of thrombi in particular in patients with AF.

A transient ischemic attack (TIA) is caused by the transient disturbance of blood supply to an area of the brain, resulting in brief neurologic dysfunction that persists usually for less than 1 hour sometimes up to 24 hours; if symptoms persist for a longer time then it is categorized as a stroke.

Transient ischemic attacks are often considered as a warning for an approaching stroke. About one third of patients with transient ischemic attack will have recurrent transient ischemic attacks and another third a stroke due to permanent nerve cell loss.

The most common cause of a transient ischemic attack is an embolus (blood clot) that occludes an artery in the brain. This can come from an atherosclerotic plaque in one of the two carotid arteries or from the heart for example in case of atrial fibrillation.

The most frequent symptoms include temporary amaurosis (loss of vision); aphasia (difficulty speaking); hemiparesis (weakness of one side of the body; paresthesia (numbness), of on one side of the body.

AF is increasingly frequent with advancing age and is often caused by age-related changes in the heart, physical or psychological stress, agents that stimulate the heart, such as caffeine, or as a result of cardiovascular disease. The number is expected to double in the next 20 years. Without appropriate management, AF can lead to serious complications, such as stroke and congestive heart failure.

As most of the studies did not assess the complications associated with atrial fibrillation such as stroke, so the effect of antiarrhythmic drugs on these endpoints is unknown (Cochrane Collaboration, The Cochrane Library, 2008, 2).

In addition, two large studies including antiarrhythmic drugs in AF patients, AFFIRM (D.G. Wyse and al., The New England Journal of Medecine, 2002, vol. 347, p.1825-1833) and AF-CHF (D. Roy and al., The New England Journal of Medecine, 2008, vol. 358, p.2667-2677), did not show a significant difference in stroke rates between the rate and rhythm groups (the recommended antiarrhythmic drug in the rhythm group was mainly amiodarone).

The Inventors have now found that dronedarone reduces the occurrence of stroke while this was not demonstrated for other antiarrhythmic compounds.

The subject of the instant invention is the use of dronedarone or one of its pharmaceutically acceptable salts for the preparation of a medicament intended to the prevention of stroke or transient ischemic attack notably in patients with a history of atrial fibrillation or atrial flutter.

The subject of the instant invention is also the use of dronedarone or one of its pharmaceutically acceptable salts for the preparation of a medicament intended to the prevention of stroke notably in patients with a history of atrial fibrillation or atrial flutter.

In contrast to cerebral circulatory insufficiency, which is a chronic disease with slowly deteriorating cognitive function a stroke is an acutely or subacutely evolving neurological deficit of cerebrovascular cause defined by symptoms that persists beyond 24 hours due to a disturbance in the blood vessels of the brain or defined by imaging of an acute clinically relevant brain lesion in patients with rapidly vanishing symptoms.

This can be due to ischemia (lack of blood supply) caused by thrombosis or embolism, or due to a haemorrhage. (R.L. Sacco et al., Stroke, 2006; vol. 37 p.577-617)

Stroke can cause permanent neurological damage or death. It is the leading cause of adult disability in the United States and Europe.

Risk factors for stroke include advanced age, hypertension, previous stroke or transient ischemic attack (TIA), diabetes, high cholesterol, cigarette smoking, atrial fibrillation, etc. Hypertension is the most important modifiable risk factor of stroke.

The symptoms of a stroke are similar to those of a transient ischemic attack but last more than 24 hours.

The main strokes are ischemic or hemorrhagic strokes. Ischemic strokes are more frequent and in some case could become hemorrhagic strokes.

In an embodiment, the invention relates to the use of dronedarone or one of its pharmaceutically acceptable salts for the preparation of a medicament intended to the prevention of ischemic stroke notably in patients with a history of atrial fibrillation or atrial flutter.

More precisely, the invention relates to the use of dronedarone or one of its pharmaceutically acceptable salts for the preparation of a medicament intended to the prevention of about 35% of stroke or transient ischemic attack in patients with a history of atrial fibrillation or atrial flutter.

More precisely, the invention relates to the use of dronedarone or one of its pharmaceutically acceptable salts for the preparation of a medicament intended to the prevention of about 35 % of stroke in patients with a history of atrial fibrillation or atrial flutter.

The percentages above correspond to an average.

Among the pharmaceutically acceptable salts of dronedarone, mention may be made of the hydrochloride.

The treated patients may be patients with a history of atrial fibrillation or atrial flutter.

The expression « with a history of atrial fibrillation or atrial flutter » means a patient who has previously manifested at least one symptom of atrial fibrillation (AF) or atrial flutter (AFL) and who can be either in sinus rhythm or in atrial fibrillation or atrial flutter at the time of dronedarone administration.

In the instant invention, "atrial fibrillation" means atrial fibrillation and/or atrial flutter.

Among patients with a history of atrial fibrillation or atrial flutter, mention may be made of patients who further have at least one of the following risk factors :

- hypertension,
- diabete,
- prior cerebrovascular accident or systemic embolism,
- left atrium diameter greater that or equal to 50 mm by echocardiography,
- left ventricular ejection fraction less than 40% by 2D-echocardiography.

Among patients with a history of atrial fibrillation or atrial flutter, mention may also be made of patients having additional risk factors corresponding to at least one of the following diseases:

- hypertension,
- structural heart disease,
- tachycardia,
- coronary heart disease,
- non-rheumatic valvular heart disease,
- ischemic dilated cardiomyopathy,
- a history of ablation for AF/AFL for example catheter ablation or surgical ablation,
- supra-ventricular tachycardia other than AF/AFL,
- history of cardiac valve surgery,
- non-ischemic dilated cardiomyopathy,
- hypertrophic cardiomyopathy,
- rheumatic valvular heart disease,
- sustained ventricular tachycardia,
- congenital heart disease,
- a history of ablation for other reason than AF/AFL for example catheter ablation,
- ventricular fibrillation,

and/or at least a cardiac device chosen among:

- a pacemaker,
- an implanted cardioverter defibrillator.

Another object of the invention is a pharmaceutical composition which comprises, as active principle, a compound of formula (I) according to the present invention. This pharmaceutical composition comprises an effective dose of at least one compound of formula (I) according to the invention, or an addition salt thereof with a pharmaceutically acceptable salt, or a hydrate or solvate thereof, and at least one pharmaceutically acceptable excipient. Said excipients are chosen according to the pharmaceutical form and the administration route desired, among usual excipients known to one of skill in the art.

In the pharmaceutical compositions according to the invention for the oral, sublingual, sub-cutaneous, intramuscular, intra-venous, topical, local, intratracheal, intranasal, transdermal or rectal administration, the active principle of formula (I) above or its salt, solvate or hydrate, can be administered as a unitary dosage form, in blend with usual pharmaceutical excipients, to animals and human beings for the prevention or for the treatment of pathological states mentioned above. The appropriate unitary dosage forms comprise the oral forms, such as tablets, hard or soft gelatin capsules, powders, granules and oral solutions or suspensions, the sublingual, buccal, intratracheal, intraocular, intranasal forms, the forms adapted to inhalation, topical, transdermal, sub-cutaneous, intramuscular or intra-venous delivery, the rectal forms and the implants. For the topical application, the compounds of the invention may be used as creams, gels, ointments or lotions.

For its use in therapeutics, dronedarone and its pharmaceutically acceptable salts are incorporated in pharmaceuticals compositions.

These pharmaceutical compositions comprise an effective dose of at least dronedarone or one of its pharmaceutically acceptable salts and at least one pharmaceutically acceptable excipient.

Said excipients are chosen according to the pharmaceutical form and the administration route desired, among usual excipients known of one of skill in the art.

In the pharmaceutical compositions for the oral, sublingual, sub-cutaneous, intramuscular, intra-venous, topical, local, intratracheal, intranasal, transdermal or rectal administration, dronedarone or one of its pharmaceutically acceptable salts, can be administered as a unitary dosage form, in blend with usual pharmaceutical excipients, to animals and human in diseases above mentioned.

The appropriate unitary dosage forms comprise the oral forms, such as tablets, hard or soft gelatin capsules, powders, granules and oral solutions or suspensions, the sublingual, buccal, intratracheal, intraocular, intranasal forms, by inhalation, the topical, transdermal, sub-cutaneous, intramuscular or intra-venous forms, the rectal forms and the implants. For the topical application, the compounds of the invention may be used as creams, gels, ointments or lotions.

As an example, a unitary dosage form for dronedarone or one of its pharmaceutically acceptable salts, in the form of a tablet, can comprise the following ingredients:

| Ingredients | mg |
| --- | --- |
| dronedarone hydrochloride (corresponding to 400 mg of base) | 426 |
| Methylhydroxypropylcellulose | 21,1 |
| Lactose monohydrate | 46,55 |
| Modified corn starch | 45,5 |
| Polyvinylpyrrolidone | 65 |
| Poloxamer 407 | 40 |
| Anhydrous colloidal silica | 2,6 |
| magnesium stearate | 3,25 |
| | 650 |

| Ingredients | mg |
| --- | --- |
| dronedarone hydrochloride (corresponding to 400 mg of base) | 426 |
| microcristalline cellulose | 65 |
| Anhydrous colloidal silica | 2,6 |
| anhydrous lactose | 42,65 |
| Polyvinylpyrrolidone | 13 |
| Poloxamer 407 | 40 |
| Macrogol 6000 | 57,5 |
| magnesium stearate | 3,25 |

(continued)

| Ingredients | mg |
|---|---|
| | 650 |

| Ingredients | mg |
|---|---|
| dronedarone hydrochloride (corresponding to 400 mg of base) | 426 |
| microcristalline cellulose | 26 |
| corn starch | 45,5 |
| Polyvinylpyrrolidone | 65 |
| Poloxamer 407 | 40 |
| Anhydrous colloidal silica | 3,25 |
| magnesium stearate | 3,25 |
| Lactose monohydrate | 41,65 |
| | 650 |

| Ingredients | mg |
|---|---|
| dronedarone hydrochloride (corresponding to 400 mg of base) | 213 |
| microcrystalline cellulose | 13 |
| corn starch | 22,75 |
| Polyvinylpyrrolidone | 32,5 |
| Poloxamer 407 | 20 |
| Anhydrous colloidal silica | 1,3 |
| magnesium stearate | 1,625 |
| Lactose monohydrate | 20,825 |
| | 650 |

For oral administration, dronedarone daily dose may reach 800 mg.

In specific cases, higher or lower dosages may be appropriate; these dosages are comprised within the scope of the present invention. According to usual practice, the dosage suitable to each patient is determined by the physician according to the administration route, the weight, the disease, the body surface, the cardiac output and response of the patient.

The instant invention also relates to a method of prevention of stroke which comprises the administration to a patient of an effective dose of at least dronedarone or one of its pharmaceutically acceptable salts.

The invention is illustrated with the above data with reference to the following figure:

Figure 1 represents Kaplan Meier cumulative incidence curves of time to first stroke or TIA according to the on-treatment analysis of 30 months;
Figure 2 represents Kaplan Meier cumulative incidence curves of time to first stroke according to the on-treatment analysis of 30 months.

Efficacy of dronedarone and its pharmaceutically acceptable salts versus placebo for the prevention of permanent atrial fibrillation was provided via dronedarone hydrochloride during a prospective, multinational, double-blind, randomized, multi-center, placebo-controlled, parallel group trial.

## I. Selection of patients

Eligible patients have a history of atrial fibrillation or atrial flutter and/or may be in normal sinus rhythm or in atrial fibrillation or flutter at the time of recruitment.

Recruitment of patients was conducted taking into account the following inclusion criteria:

Inclusion criteria :

1) One of the following risk factors must be present :

- Age equal or greater that 70 years, or above 75 years, associated or not with at least one of the following risk factors :

    o hypertension (taking antihypertensive drugs of at least two different classes),
    o diabetes,
    o prior cerebrovascular accident (stroke or transient ischemic attack) or systemic embolism,
    o left atrium diameter greater that or equal to 50 mm by echocardiography,
    o left ventricular ejection fraction less than 40% by 2D-echocardiography,

2) availability of one electrocardiogram within the last six months, showing that the patients was or is in atrial fibrillation/flutter,

3) availability of one electrocardiogram within the last six months, showing that the patients was or is in sinus rhythm.

## II. Duration and treatment

Study drug treatment units (placebo or dronedarone hydrochloride corresponding to 400 mg of base) were such that each patient took one tablet in the morning during or shortly after breakfast and one tablet in the evening during or shortly after dinner.

The treatment duration depended on the time of recruitment of each patient in the trial and could be comprised from 12 months to 30 months.

## III. Results

Results were calculated using non-parametric Kaplan-Meier estimate.

Cox's proportional hazard model was used to estimate the hazard ratio also called relative risk.

Relative risk (RR) is the ratio between the risk of having a stroke (or transient ischemic attack (TIA)) for patients treated with dronedarone and the risk of having a stroke (or transient ischemic attack (TIA)) for patients treated with placebo.

Percentage of decrease of an event is calculated as follow:

$$x = 1 - RR.$$

Results relating to the prevention of stroke or transient ischemic attack (TIA)

From the 4628 patients included in the trial, 2301 were part of the group treated with dronedarone hydrochloride.

80 stroke or TIA events were reported in the placebo group versus 52 in the group treated with dronedarone hydrochloride.

Calculated relative risk was equal to 0.65, i.e. a decrease of the relative risk of stroke or TIA of 35%.

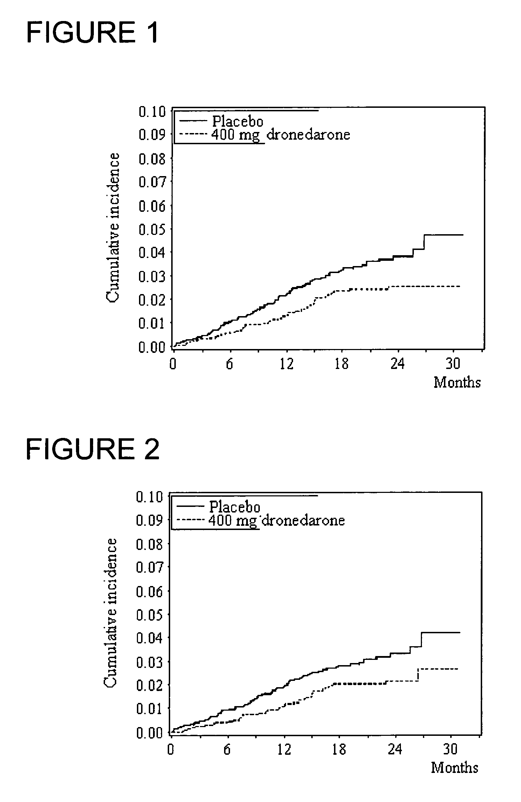

Figure 1 shows that the effect of dronedarone occurred early and increased over time.

Results relating to the prevention of stroke

From the 4628 patients included in the trial, 2301 were part of the group treated with dronedarone hydrochloride.

70 stroke events were reported in the placebo group versus 45 in the group treated with dronedarone hydrochloride.
Calculated relative risk was equal to 0.65, i.e. a decrease of the relative risk of stroke of 35%.
Figure 2 shows that the effect of dronedarone occurred early and increased over time.

Results relating to the prevention of ischemic stroke

From the 4628 patients included in the trial, 2301 were part of the group treated with dronedarone hydrochloride.
49 stroke events were reported in the placebo group versus 33 in the group treated with dronedarone hydrochloride.

**Claims**

1. Use of dronedarone for the preparation of a medicament intended for the prevention of stroke or transient ischemic attack.

2. Use according to claim 1 for the preparation of a medicament intended for the prevention of stroke.

3. Use according to claim 1 for the preparation of a medicament intended for the prevention of about 35 % of stroke.

4. Use according to claim 1, 2 or 3, **characterized in that** the patients have a history of atrial fibrillation or atrial flutter.

5. Use according to one of the preceding claims, **characterized in that** the patients have at least one of the following risk factors :

   - hypertension,
   - diabete,
   - prior cerebrovascular accident or systemic embolism,
   - left atrium diameter greater that or equal to 50 mm by echocardiography,
   - left ventricular ejection fraction less than 40% by 2D-echocardiography.

6. Use according to 1, 2, 3 or 4, **characterized in that** the patients have additional risk factors corresponding to at least one of the following diseases:

   - hypertension,
   - structural heart disease,
   - tachycardia,
   - coronary heart disease,
   - non-rheumatic valvular heart disease,
   - ischemic dilated cardiomyopathy,
   - ablation for AF/AFL,
   - supra-ventricular tachycardia other than AF/AFL,
   - history of cardiac valve surgery,
   - non-ischemic dilated cardiomyopathy,
   - hypertrophic cardiomyopathy,
   - rheumatic valvular heart disease,
   - sustained ventricular tachycardia,
   - congenital heart disease,
   - ablation for other reason than AF/AFL,
   - ventricular fibrillation,

   and/or at least a cardiac device chosen among:

   - a pacemaker,
   - an implanted cardioverter defibrillator.

7. Use according to one of the preceding claims, **characterized in that**, for oral administration, dronedarone daily dose may reach 800 mg.

8. Method of prevention of stroke which comprises the administration to a patient of an effective dose of at least dronedarone or one of its pharmaceutically acceptable salts.

# FIGURE 1

# FIGURE 2

**EUROPEAN SEARCH REPORT**

Application Number

EP 08 29 0761

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SCHWENDER THERESE: "La dronédarone rétablit et maintient le rythme sinusal [Dronedarone establishes and maintains control of sinus rhythm]" REVUE MÉDICALE SUISSE, MÉDECINE & HYGIÈNE, GENEVA, CH, vol. 2, no. 76, 23 August 2006 (2006-08-23), pages 1902-1903, XP008096444 ISSN: 1660-9379 * abstract; figure 1 * * page 1902, right-hand column, lines 9-14, paragraph 1 * * page 1903, left-hand column, paragraph 2 * | 1-8 | INV. A61K31/343 A61P9/10 |
| X | CAMM ET AL: "Safety considerations in the pharmacological management of atrial fibrillation" INTERNATIONAL JOURNAL OF CARDIOLOGY, ELSEVIER SCIENCE PUBLISHERS, AMSTERDAM, NL, vol. 127, no. 3, 21 July 2008 (2008-07-21), pages 299-306, XP022794944 ISSN: 0167-5273 [retrieved on 2008-01-08] * page 299, left-hand column, line 1, paragraph 2 - page 299, right-hand column, line 2, paragraph 1 * * page 303, left-hand column, lines 1-4, paragraph 2 * | 1-8 | TECHNICAL FIELDS SEARCHED (IPC)  A61K |

-/--

The present search report has been drawn up for all claims

| Place of search | Date of completion of the search | Examiner |
|---|---|---|
| Munich | 14 January 2009 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 08 29 0761

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAVY J -M ET AL: "The efficacy and safety of dronedarone as a novel rate control agent for the treatment of atrial fibrillation" EUROPEAN HEART JOURNAL, vol. 26, no. Suppl. 1, September 2005 (2005-09), pages 505-P3042, XP002509809 & 27TH CONGRESS OF THE EUROPEAN-SOCIETY-OF-CARDIOLOGY; STOCKHOLM, SWEDEN; SEPTEMBER 03 -07, 2005 ISSN: 0195-668X * abstract * | 1-8 | |
| Y | | 1-8 | |
| | ----- | | |
| X | "Dronedarone: drondarone, SR 33589, SR 33589B" DRUGS IN R & D, ADIS INTERNATIONAL, AUCKLAND, NZ, vol. 8, no. 3, 1 January 2007 (2007-01-01), pages 171-175, XP008096407 ISSN: 1174-5886 * abstract * * page 174, right-hand column, paragraph 3 - page 175, left-hand column, paragraph 1 * | 1-8 | |
| Y | | 1-8 | TECHNICAL FIELDS SEARCHED (IPC) |
| | ----- | | |
| | -/-- | | |

The present search report has been drawn up for all claims

| Place of search | Date of completion of the search | Examiner |
|---|---|---|
| Munich | 14 January 2009 | Madalinska, K |

EPO FORM 1503 03.82 (P04C01)

**EP 2 153 830 A1**

Europäisches
Patentamt

European
Patent Office

Office européen
des brevets

# EUROPEAN SEARCH REPORT

Application Number

EP 08 29 0761

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PURERFELLNER H: "ATHENA Studie: Vorläufige Ergebnisse und deren mögliche Auswirkungen auf die antiarrhythmische Pharmakotherapie bei Vorhofflimmern [ATHENA study: Preliminary results and their possible effects on the antiarrhythmic pharmacotherapy in atrial fibrillation]" JOURNAL FUER KARDIOLOGIE, KRAUSE UND PACHERNEGG, PURKERSDORF, AT, vol. 15, no. 7-8, 1 January 2008 (2008-01-01), pages 253-254, XP008096461 ISSN: 1024-0098 * the whole document * ----- | 1-8 | |
| X | WHITE C MICHAEL: "Is dronedarone effective for the prevention of recurrent atrial fibrillation?" NATURE CLINICAL PRACTICE CARDIOVASCULAR MEDICINE, NATURE PUBLISHING GROUP, LONDON, GB, vol. 5, no. 3, 1 March 2008 (2008-03-01), pages 136-137, XP008096446 ISSN: 1743-4297 * the whole document * ----- -/-- | 1-8 | TECHNICAL FIELDS SEARCHED (IPC) |

The present search report has been drawn up for all claims

| Place of search | Date of completion of the search | Examiner |
|---|---|---|
| Munich | 14 January 2009 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

12

| | Europäisches Patentamt European Patent Office Office européen des brevets | EUROPEAN SEARCH REPORT | Application Number EP 08 29 0761 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOHNLOSER STEFAN H ET AL: "Dronedarone significantly decreases the combined endpoint of hospitalization and death in patients with atrial fibrillation" CIRCULATION, AMERICAN HEART ASSOCIATION, DALLAS, TX, vol. 112, no. 17, Suppl. S, 1 October 2005 (2005-10-01), pages U383-U384, XP008096460 ISSN: 0009-7322 * abstract * | 1-8 | |
| Y | | 1-8 | |
| Y | WO 91/16045 A (SMITH KLINE FRENCH LAB [GB]) 31 October 1991 (1991-10-31) * abstract * * page 2, lines 9-23 * | 1-8 | |
| T | STILES S.: "Athena analysis suggests cut in stroke risk with dronedarone for atrial fib" THE HEART.ORG, [Online] 3 September 2008 (2008-09-03), XP002509840 Retrieved from the Internet: URL:http://www.theheart.org/article/print. do?primaryKey=901685> [retrieved on 2009-01-12] * the whole document * | 1-8 | TECHNICAL FIELDS SEARCHED (IPC) |

The present search report has been drawn up for all claims

| Place of search | Date of completion of the search | Examiner |
|---|---|---|
| Munich | 14 January 2009 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 08 29 0761

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2009

| Patent document cited in search report | | Publication date | Patent family member(s) | | Publication date |
|---|---|---|---|---|---|
| WO 9116045 | A | 31-10-1991 | AT | 181668 T | 15-07-1999 |
| | | | AU | 653521 B2 | 06-10-1994 |
| | | | AU | 7768091 A | 11-11-1991 |
| | | | CA | 2081344 A1 | 27-10-1991 |
| | | | DE | 69131398 D1 | 05-08-1999 |
| | | | DE | 69131398 T2 | 02-03-2000 |
| | | | DK | 526540 T3 | 22-11-1999 |
| | | | EP | 0526540 A1 | 10-02-1993 |
| | | | ES | 2134774 T3 | 16-10-1999 |
| | | | GR | 3031042 T3 | 31-12-1999 |
| | | | IE | 911383 A1 | 06-11-1991 |
| | | | JP | 5509293 T | 22-12-1993 |
| | | | JP | 2003267890 A | 25-09-2003 |
| | | | PT | 97477 A | 31-01-1992 |
| | | | ZA | 9103071 A | 25-11-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0471609 B1

**Non-patent literature cited in the description**

- **D.G. Wyse.** *The New England Journal of Medecine,* 2002, vol. 347, 1825-1833
- **D. Roy.** *New England Journal of Medecine,* 2008, vol. 358, 2667-2677
- **R.L. Sacco et al.** *Stroke,* 2006, vol. 37, 577-617